# EUROPÄISCHE PATENTSCHRIFT

(11) **EP 2 453 805 B1**
(45) Veröffentlichungstag und Bekanntmachung des Hinweises auf die Patenterteilung: **10.09.2014**
(21) Anmeldenummer: 10737767.3
(22) Anmeldetag: 13.07.2010
(51) Int. Cl.: A61B 17/00, A61B 17/11

(54) **VORRICHTUNG ZUR HERSTELLUNG VON ANASTOMOSEN**
DEVICE FOR PRODUCING ANASTOMOSES
DISPOSITIF PERMETTANT DE RÉALISER DES ANASTOMOSES

(30) Priorität: 14.07.2009 DE 102009032972
(43) Veröffentlichungstag der Anmeldung: 23.05.2012
(73) Patentinhaber: ERBE Elektromedizin GmbH, 72072 Tübingen (DE)
(72) Erfinder: BRODBECK, Achim, 72555 Metzingen (DE)
(74) Vertreter: Rüger, Barthelt & Abel
(86) Internationale Anmeldenummer: PCT/EP2010/004259
(87) Internationale Veröffentlichungsnummer: WO 2011/006639

(56) Entgegenhaltungen:
- WO-A1-03/061487
- WO-A1-2007/030892

## Beschreibung

Die Erfindung betrifft eine Vorrichtung zum Herstellen von Anastomosen gemäß dem Patentanspruch 1.

Eine Anastomose ist eine Verbindung zwischen zwei anatomischen Strukturen. Beispielsweise können durchtrennte Blutgefäße bei einer Organtransplantation mittels Anastomosen verbunden werden.

In der Chirurgie sind unterschiedliche Verfahren zur Herstellung derartiger Anastomosen bekannt. Die Verbindung kann unter Verwendung von Nahttechniken hergestellt werden. Des Weiteren existieren Ansätze, die eine organische Verbindung mittels Klebetechnik erlauben. Hierbei werden häufig Fibrinkleber eingesetzt, die eine sehr vorteilhafte Verbindung von Organen, insbesondere Hohlorganen ermöglichen. Problematisch ist jedoch die Verträglichkeit dieser Kleber bzw. Klebstoffe, da sie thrombogene und toxische Eigenschaften aufweisen können.

Klemmtechniken werden ebenfalls eingesetzt, um an geeigneter Stelle eine Verbindung herzustellen. Zur Unterstützung der Durchführung von Anastomosen werden verschiedene Hilfsmittel wie Ringe, Manschetten (so genannte Cuffs) oder Stents verwendet. Nachteilig bei der Anwendung dieser Klemmtechniken ist es, dass die genannten Hilfsmittel in der Regel in dem zu verbindenden Hohlorgan verbleiben und dort Abstoßreaktionen hervorrufen können. Auch hier droht die Gefahr einer Thrombosebildung.

Aus der WO 03/061487 A1 ist es bekannt, Hohlorgane durch die Applikation eines geeigneten Hochfrequenzstroms (HF-Stroms) zu verbinden. Bei der Applikation des HF-Stroms kommt es zu einem Verschweißen der Gewebestrukturen. Die Zellsubstanz gerinnt, wobei die Proteinstrukturen verkleben, so dass eine kontrollierte, sichere und schnelle Verbindung hergestellt werden kann. Zur Applikation des HF-Stroms beschreibt die WO 03/061487 A1 ein Instrument mit einer inneren Hülse und einer äußeren Hülse. Diese Hülsen umfassen jeweils eine Elektrode, die ringförmig ausgebildet ist und an die eine geeignete HF-Spannung angelegt werden kann. Es handelt sich also um ein bipolares elektrochirurgisches Instrument. Zur Verbindung der Enden oder Endabschnitte eines Blutgefäßes wird das erste Ende durch die Innenhülse geführt und derart umgeschlagen, dass das Gewebe auf der Außenseite der inneren Hülse zu liegen kommt. Das zweite Ende des Gefäßes wird über die innere Hülse und den darauf befindlichen Endabschnitt des Blutgefäßes gezogen. Die äußere Hülse lässt sich öffnen und über die innere Hülse und das darauf befindliche Gewebe stülpen. Die Außenhülse bildet also eine Art Manschette, die die einzelnen Endabschnitte des Blutgefäßes umschließt. Sowohl die innere Hülse als auch die äußere Hülse weisen eine Elektrode zur Applikation des HF-Stroms auf. Diese Elektroden liegen einander gegenüber. Bei einer Applikation des HF-Stroms durchströmt dieser das Gewebe - also die übereinander liegenden Endabschnitte - und verschweißt das Gefäß. Ein Problem der in der WO 03/061487 A1 beschriebenen Technik besteht darin, dass die hergestellten Verbindungen häufig nicht ausreichend stabil sind.

Die WO 2007/030892 beschreibt ein Verfahren zum Verbinden zweier Hohlorgane, wobei ein Röhrenimplantat aus einer im Wesentlichen festen Komposition aus Biomolekülen über das Ende eines Hohlorgans gezogen wird. Das Ende des Hohlorgans wird sodann über das Röhrenimplantat gestülpt und das Röhrenimplantat wird mit dem Hohlorgan verklebt. Das Ende des zweiten Hohlorgans wird über den umgestülpten Teil des ersten Hohlorgans bis über das Röhrenimplantat gezogen und mit dem Röhrenimplantat verklebt.

Ausgehend von diesem Stand der Technik ist es Aufgabe der vorliegenden Erfindung, eine Vorrichtung zur Herstellung einer Anastomose zwischen einem ersten und einem zweiten Hohlorgan bereitzustellen, die einfach zu bedienen und zur Bildung von qualitativ hochwertigen Anastomosen geeignet ist.

Diese Aufgabe wird durch die Vorrichtung gemäß Anspruch 1 gelöst.

Insbesondere wird die Aufgabe durch eine Vorrichtung zur Herstellung einer oder mehrerer Anastomosen zwischen einem ersten Hohlorgan und einem zweiten Hohlorgan gelöst, wobei die Hohlorgane jeweils eine Außenfläche und eine Innenfläche haben, wobei die Vorrichtung umfasst:
- eine Hülse, über welche das erste Hohlorgan so umgestülpt werden kann, dass mindestens ein Klebeverbundabschnitt der Innenfläche des ersten Hohlorgans nach außen zu liegen kommt und auf weicher das zweite Hohlorgan über dem ersten Hohlorgan positioniert werden kann;
- einen Klebeapplikator, der zur Applikation eines Klebstoffs auf den Klebeverbundabschnitt neben der Hülse positionierbar ist.

Erfindungsgemäß soll also eine Vorrichtung bereitgestellt werden, die einen Klebeapplikator umfasst, der die Applikation des Klebstoffs vereinfacht. Die Hülse dient hierbei als Haltevorrichtung, um zumindest das erste Hohlorgan, beispielsweise ein Blutgefäß so zu fixieren und/oder positionieren, dass der Klebstoff appliziert werden kann. Für die Applikation des Klebstoffs wird zumindest ein Teil des Klebeapplikators neben der Hülse positioniert. Dieser Teil kann eine Düse oder ein Auslass sein, der so, vorzugsweise gegenüber, dem Verbundabschnitt oder Klebeverbundabschnitt angeordnet werden kann, dass eine vorzugsweise unmittelbare Applikation des Klebstoffs hierauf erfolgen kann. Die Applikation des Klebstoffs erfolgt vorzugsweise auf einen Abschnitt der umgeschlagenen Innenfläche des ersten Hohlorgans, nämlich dem Verbundabschnitt. Dieser Verbundabschnitt wird dann in einem späteren Schritt vorzugsweise mit einem Abschnitt der Innenfläche des zweiten Hohlorgans verklebt. Erfindungsgemäß kann mittels der Vorrichtung erst der Klebstoff auf das erste Hohlorgan appliziert werden und dann das zweite Hohlorgan über das erste Hohlorgan übergezogen werden. In einem anderen Ausführungsbeispiel können das erste und zweite Hohlorgan erst positioniert werden und dann der Klebstoff an dem Verbundabschnitt appliziert werden. Die Vorrichtung ermöglicht eine örtlich exakt positionierte Applikation des Klebstoffs.

Die Vorrichtung kann eine Führung umfassen, in der der Klebeapplikator oder Teile des Klebeapplikators zwischen einer Applikationsposition zur Applikation des Klebstoffs und einer Aufnahmeposition zur Aufnahme mindestens des ersten Hohlorgans durch die Hülse verschiebbar angeordnet ist. Für das Einführen des ersten Hohlorgans in die Hülse und das Umstülpen ist es notwendig, dass ausreichend Platz zur Verfügung steht. Deswegen ist es vorteilhaft, wenn insbesondere störende Teile des Klebeapplikators verschiebbar angeordnet sind, so dass diese beim Aufnehmen des Hohlorgans ausreichend von der Hülse beabstandet sind.

In der Applikationsposition kann das erste Hohlorgan zwischen dem Klebeapplikator und der Hülse fixiert sein. Es ist also vorteilhaft, wenn die Führung des Klebeapplikators derart ausgebildet ist, dass in der Applikationsposition zumindest Teile des Klebeapplikators als Fixierung dienen. Diese Teile können zumindest das erste Hohlorgan zwischen dem Klebeapplikator und der Hülse fixieren, so dass das Hohlorgan nicht aus der Vorrichtung rutschen kann.

Der Klebeapplikator kann mindestens einen Klebekanal, insbesondere eine Klebekapillare, umfassen, wobei zumindest ein Endabschnitt des Klebekanals zum Einbringen des Klebstoffs zwischen dem ersten und dem zweiten Hohlorgan im Wesentlichen parallel zur Hülse angeordnet ist oder dort angeordnet werden kann. Vorzugsweise wird also der Klebstoff nach der Positionierung des ersten und zweiten Hohlorgans in einen überlappenden Bereich zwischen diesen Organen eingebracht. Vorzugsweise handelt es sich hierbei um eine Überlappung der Innenflächen der beiden Hohlorgane.

Die Vorrichtung kann mindestens zwei Klebekanäle aufweisen, die einen definierten Abstand zueinander haben und wechselseitig an der Hülse positionierbar sind. Es ist vorteilhaft, wenn mehrere Klebekanäle bereitstehen, die den Klebstoff an unterschiedlichen Seiten der Hülse applizieren können. Vorzugsweise handelt es sich hierbei um zwei Klebekanäle, die wechselseitig der Hülse positionierbar sind. Für die Positionierung kann die Führung entsprechend ausgebildet sein.

Der mindestens eine Klebekanal kann ein abgeflachtes Ende zum Applizieren des Klebstoffs umfassen. Das heißt, der Endabschnitt des Klebekanals, der zur Applikation des Klebstoffs auf das Gewebe, insbesondere auf die Innenfläche des ersten Hohlorgans dienen soll, ist vorzugsweise abgeflacht, so dass das zweite Hohlorgan problemlos übergestreift werden kann. Für die Herstellung der Anastomosen ist eine Vorrichtung vorzuziehen, die es ermöglicht, erst das erste Hohlorgan aufzunehmen, danach den Klebeapplikator an entsprechender Stelle vorzusehen und danach das zweite Hohlorgan zu positionieren. Somit kann darauf verzichtet werden, den Klebeapplikator zwischen den Hohlorganen aufwändig einzuführen. Die Position des Klebeapplikators kann visuell überwacht werden.

Der Klebekanal kann mindestens einen Anschlag zur Positionierung des zweiten Hohlorgans oder eines Abschnitts dessen in Längsrichtung der Hülse umfassen. Die Längsrichtung der Hülse ist durch die beiden Öffnungen, die den Hülsenkanal miteinander verbinden und das erste Hohlorgan aufnehmen, definiert. Nach der Positionierung des ersten Hohlorgans kann das Positionieren des zweiten Hohlorgans problematisch sein. Vorzugsweise bietet der Klebeapplikator einen Anschlag an, der beim Überziehen des zweiten Hohlorgans über die Hülse und über das erste Hohlorgan eine schlechte Positionierung (die offenen Enden der Hohlorgane liegen nicht parallel zueinander) verhindert.

Der Klebekanal kann einen Winkelabschnitt, insbesondere mit einem Innenwinkel ≤ 90°, als Anschlag für das zweite Hohlorgan haben. Vorzugsweise weist der mindestens eine Klebekanal also eine Verformung auf, an der das zweite Hohlorgan positioniert werden kann.
Die Hülse kann an einer Betätigungseinrichtung gehalten und derart in mindestens zwei Teile zerlegbar ausgebildet sein, dass die Teile von einem Schließzustand zur Bildung eines im Wesentlichen geschlossenen Rohrabschnitts in einen Öffnungszustand zum Abnehmen des verbundenen Hohlorgans bringbar sind. Bereits bei der Aufnahme des ersten Hohlorgans kann es vorteilhaft sein, wenn die Hülse zerlegbar ist. Nach einer Verbindung der beiden Hohlorgane zu einem Hohlorgan muss die Hülse entweder zerlegbar oder unterbrochen sein, um die Vorrichtung von dem Hohlorgan abzunehmen.

Die Vorrichtung kann einen Kompressionsring umfassen, der die Hülse zumindest abschnittsweise umgebend angeordnet ist oder dort angeordnet werden kann, um in einer Kompressionsposition die Hohlorgane zwischen dem Kompressionsring und der Hülse zu halten. Der Kompressionsring hat also zum einen den Vorteil, dass er die Hohlorgane an der Hülse festhält. Zum anderen kann er derart angeordnet und ausgebildet sein, dass er die Hohlorgane derart dicht aneinander hält, dass durch einen Kompressionsspalt zwischen den Hohlorganen kein Klebstoff durchdringen kann. Somit kann ein Aufnahmebereich für den Klebstoff geschaffen werden, der gegenüber dem Innenbereich der Hohlorgane abgedichtet ist.

Vorzugsweise handelt es sich bei der Vorrichtung um eine elektrochirurgische Vorrichtung, die Elektroden aufweist. Die Hülse kann eine Innenelektrode und der Kompressionsring eine Außenelektrode zur Applikation eines HF-Stroms umfassen. Vorzugsweise wird also mit der Vorrichtung eine erste Verbindung aufgrund des applizierten HF-Stroms und eine zweite Verbindung aufgrund des Klebstoffs hergestellt. Es wurde festgestellt, dass bipolare Gefäßanastomosen in Anlehnung an die Payr'sche Cuff-Technik nur eine begrenzte Zugbelastbarkeit aufweisen. Um diesen Nachteil auszuräumen ist es erfindungsgemäß vorgesehen, eine zusätzliche Klebeverbindung anzubringen, die weitere Abschnitte, insbesondere im Bereich der Endabschnitte der Hohlorgane verbindet und somit die Festigkeit der Gesamtverbindung erhöht. Der HF-Strom kann mittels des Kompressionsrings appliziert werden. Nach einer Applikation des HF-Stroms und einer ersten durchgehenden Verbindung der Organe hierdurch, kann der Klebstoff eingebracht werden. Die hergestellte Schweißverbindung dient als Begrenzung, so dass sich der Klebstoff nicht in das zu verbindende Organ ausbreitet.

Vorzugsweise ist der Klebeapplikator abnehmbar mit der Vorrichtung verbunden. Somit kann der Umgang mit der Vorrichtung erleichtert werden. Es ist denkbar, bekannte HF-Instrumente durch den erfindungsgemäßen Klebeapplikator zu erweitern. Des Weiteren kann der möglicherweise störende Klebeapplikator erst dann an der Vorrichtung angebracht werden, wenn dieser benötigt wird. Beispielsweise kann ein Anbringen des Klebeapplikators erst dann als sinnvoll angesehen werden, wenn bereits eine Schweißverbindung mittels des HF-Stroms zwischen den Organen hergestellt wurde.

Der Klebeapplikator kann eine Zuleitung aus nicht-adhäsivem Material, insbesondere aus Polytetrafluorethylen (PTFE) umfassen. Eine entsprechende Materialwahl kann verhindern, dass der Klebstoff die Zuleitung verstopft.

Der Klebeapplikator kann ein Klebstoffreservoir und einen Kompressor zur Applikation des Klebstoffs umfassen. Der Kompressor dient hierbei dazu, ein Treibmittel bereitzustellen, mittels dessen der Klebstoff beispielsweise durch die Zuleitung getrieben wird, so dass er an geeigneter Stelle der Hohlorgane aufgebracht werden kann.

Der Kompressor kann eine Spritze sein.

Die eingangs genannte Aufgabe wird ebenfalls durch ein beispielhaftes Verfahren zur Herstellung von Anastomosen zwischen einem ersten Hohlorgan und einem zweiten Hohlorgan gelöst, wobei die Hohlorgane jeweils eine Innenfläche haben. Das erfindungsgemäße Verfahren kann ein Umstülpen des ersten Hohlorgans über eine Hülse umfassen, so dass mindestens ein Klebeverbundabschnitt der Innenfläche des ersten Hohlorgans nach außen zu liegen kommt. Des Weiteren kann das Verfahren ein Positionieren des zweiten Hohlorgans über dem ersten Hohlorgan und ein Herstellen einer ersten Verbindung zwischen den Hohlorganen vorzugsweise durch das Anlegen einer HF-Spannung umfassen. Abschließend kann ein Herstellen einer zweiten Verbindung zwischen den Hohlorganen durch ein Applizieren eines Gewebeklebstoffs erfolgen.

Vorzugsweise wird der Gewebeklebstoff zwischen den übereinander liegenden Hohlorganen eingebracht, wobei der Klebeverbundabschnitt zumindest abschnittsweise mit dem Gewebeklebstoff bedeckt wird. Insofern wird bei dem erfindungsgemäßen Verfahren eine doppelte Verbindung zwischen dem Hohlorgan hergestellt. Die erste Verbindung beruht auf elektrochirurgischer Koagulation, während die zweite Verbindung eine chemisch hergestellte ist. Aufgrund dieser doppelten Verbindung ist die hergestellte Anastomose wesentlich belastbarer, insbesondere gegenüber Zugkräften.

Das Herstellen der ersten Verbindung kann ein Anlegen der HF-Spannung an die Hülse, insbesondere eine Innenelektrode der Hülse, und eine Außenelektrode umfassen. Vorzugsweise kontaktiert die Außenelektrode mindestens eines der Hohlorgane, so dass ein applizierter HF-Strom die Hohlorgane durchdringt und miteinander verbindet.

Das Verfahren kann ein Anlegen eines Kompressionsrings und ein Applizieren einer vordefinierten Kraft mittels des Kompressionsrings umfassen. Der Kompressionsring kann dazu verwendet werden, die Hohlorgane auf der Hülse zu fixieren, so dass diese in der vorgegebenen Position gehalten werden. Des Weiteren kann der Kompressionsring dazu eingesetzt werden, eine vordefinierte Kraft zu applizieren, die das Herstellen der ersten Verbindung unterstützt. Weiterhin kann der Kompressionsring sicherstellen, dass beim Herstellen der zweiten Verbindung der Gewebeklebstoff nicht in das Innere/Lumen des bzw. der Hohlorgane gelangt.

Vorzugsweise kann die Herstellung der zweiten Verbindung anhand eines Einspritzens des Gewebeklebstoffs in einen Zwischenraum zwischen den Hohlorganen erfolgen. Beim Einspritzen des Gewebeklebstoffs ist der Kompressionsring besonders hilfreich, da er die Hohlorgane flüssigkeitsdicht gegenüber dem Außenbereich aneinanderhält.

Das Herstellen der zweiten Verbindung kann nach dem Herstellen der ersten Verbindung erfolgen. Insofern kann der oben genannte Effekt besonders positiv ausgenutzt werden.

Die erste Verbindung kann derart hergestellt werden, dass ein Klebebereich zwischen dem ersten und dem zweiten Hohlorgan zur Aufnahme des Gewebeklebstoffs verbleibt, wobei der Klebebereich gegenüber einem Innenbereich der Hohlorgane flüssigkeitsdicht abgeschlossen ist.

Der Klebebereich kann ein Bereich zwischen den Hohlorganen sein, der sich distal der ersten Verbindung (also auf der Seite, an der die Hohlorgane offen sind) befindet. Dieser Klebebereich kann beispielsweise den bereits genannten Klebeverbundsabschnitt umfassen. Die mittels der HF-Koagulation hergestellte Verbindung versiegelt das Hohlorgan derart, dass der Klebstoff nicht in das Lumen eindringen kann. Dies kann sogar dann gewährleistet werden, wenn der Klebstoff in den genannten Klebebereich mit relativ hohem Druck eingespritzt wird.

Weitere vorteilhafte Ausführungsformen ergeben sich anhand der Unteransprüche.

Nachfolgend wird die Erfindung anhand von einem Ausführungsbeispiel beschrieben, das mittels Abbildungen näher erläutert wird. Hierbei zeigen:
- - Fig. 1: einen Klebeapplikator;
- - Fig. 2: das distale Ende des Klebeapplikators aus Fig. 1 in einer ersten Seitenansicht;
- - Fig. 3: das distale Ende des Klebeapplikators aus Fig. 1 in einer zweiten Seitenansicht;
- - Fig. 4 u. 5: ein elektrochirurgisches Instrument zur Herstellung von Anastomosen mit dem Klebeapplikator aus Fig. 1, wobei der Klebeapplikator am Instrument positioniert wird;
- - Fig. 6: das Instrument zur Herstellung von Anastomosen gemäß Fig. 4 und 5 mit positioniertem Klebeapplikator;
- - Fig. 7: das Instrument zur Herstellung von Anastomosen gemäß den Fig. 4 bis 6 mit geöffneter Kompressionszange;
- - Fig. 8: die Vorrichtung gemäß Fig. 7 mit geschlossener Kompressionszange;
- - Fig. 9: einen schematischen Längsschnitt durch ein verbundenes Organ innerhalb des Instruments zur Herstellung von Anastomosen; und
- - Fig. 10: den Längsschnitt aus Fig. 9, wobei das Instrument abgenommen wird.

In der nachfolgenden Beschreibung werden für gleiche und gleich wirkende Teile dieselben Bezugsziffern verwendet.

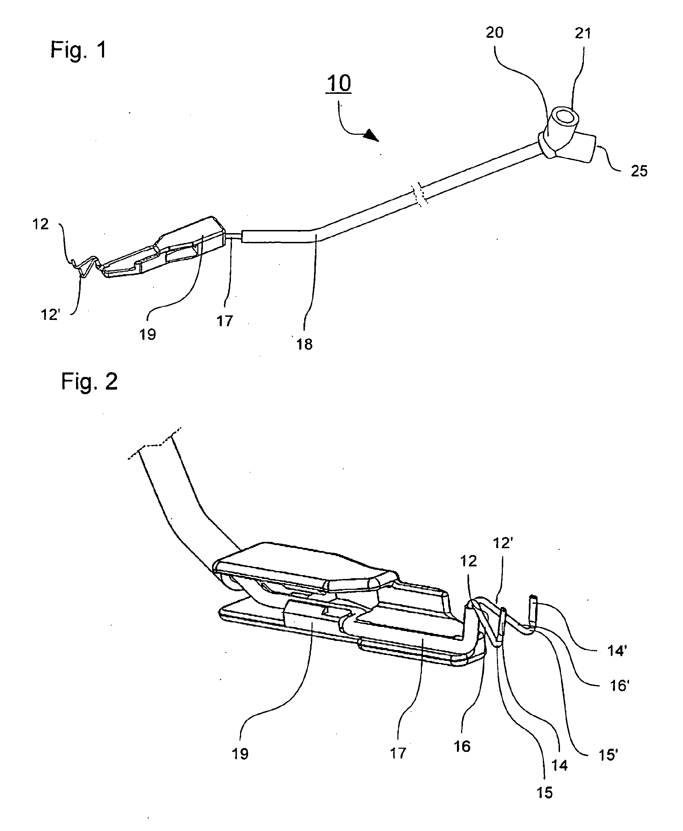

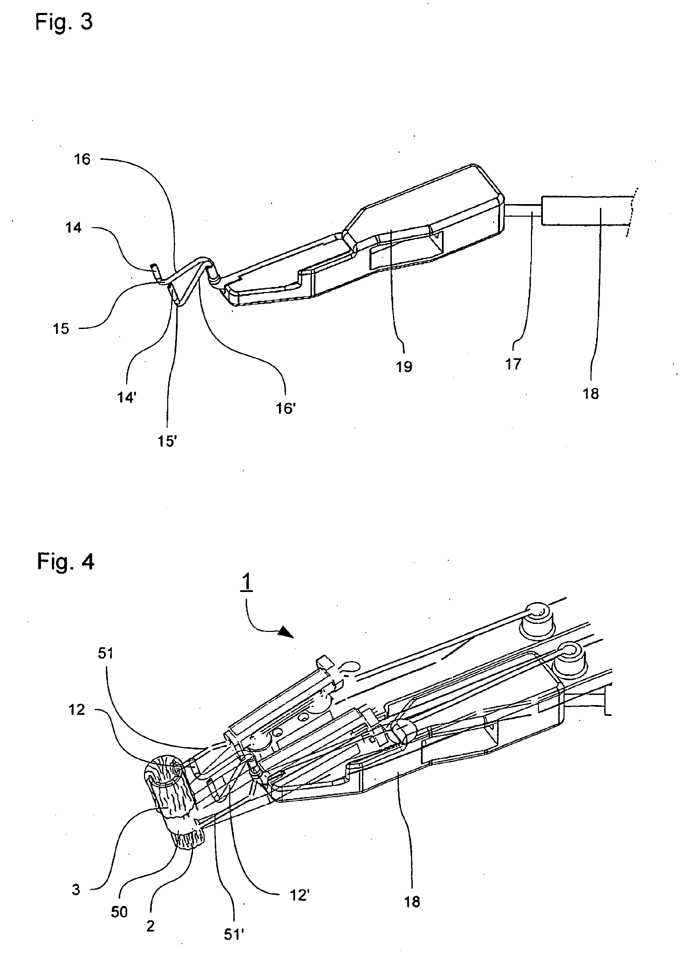

Fig. 1 zeigt einen erfindungsgemäßen Klebeapplikator 10, der sich abnehmbar mit einer Vorrichtung zur Herstellung von Anastomosen, z. B. einem elektrochirurgischen Instrument 1 (Fig. 4 bis 8), verbinden lässt.

Der Klebeapplikator 10 umfasst einen Adapter 19 mit Klebekapillaren 12, 12', ein Zuleitungsrohr 18 und ein Anschlussstück 20. Das Anschlussstück 20 bildet das proximale Ende des Klebeapplikators 10, wobei dieses über das Zuleitungsrohr 18 und eine Zuleitung 17 mit den Klebekapillaren 12, 12' verbunden ist. Das Anschlussstück 20 ist Y-förmig ausgebildet und hat einen Klebstoffanschluss 21 und einen Treibmittelanschluss 25. Über den Klebstoffanschluss 21 kann ein Klebstoff in das Zuleitungsrohr 18 eingebracht werden. Ein Treibmittel, das zeitlich verzögert über den Treibmittelanschluss 25 in das Zuleitungsrohr 18 gepumpt wird, treibt den Klebstoff durch das Zuleitungsrohr 18 in Richtung auf das distale Ende des Klebeapplikators 10 zu. Vorzugsweise sind die Klebekapillaren 12, 12' und/oder die Zuleitung 17 aus Edelstahl und das Zuleitungsrohr 18 aus nicht-adhäsivem Material, also etwa aus PTFE gefertigt. Das Zuleitungsrohr 18 ist vorzugsweise flexibel und nicht länger als 50 cm lang. Vorzugsweise hat das Zuleitungsrohr 18 eine Länge von 10 cm bis 30 cm. Aufgrund der Ausgestaltung, Materialwahl und Länge des Zuleitungsrohrs 18, der Zuleitung 17 und der Klebekapillaren 12, 12' wird eine Adhäsion innerhalb des Klebeapplikators verhindert.

Ein an den Klebstoffanschluss 21 angeschlossenes Klebstoffreservoir (nicht gezeigt) kann eine Tube mit Klebstoff sein. Ein an den Treibmittelanschluss 25 angeschlossener Kompressor oder eine entsprechende Kompressoreinrichtung kann beispielsweise eine handelsübliche medizinische Spritze sein.

Wie in den Fig. 2 und 3 gezeigt, mündet das Zuleitungsrohr 18 distal in die Zuleitung 17, die durch den Adapter 19 geführt ist. Am distalen Ende der Zuleitung 17 mündet diese in die erste Klebekapillare 12, und die zweite Klebekapillare 12'. Die Zuleitung 17 verzweigt sich also in die beiden Klebekapillaren 12, 12'. Der letzte Abschnitt der Zuleitung 17 verläuft im Wesentlichen senkrecht zur Längsrichtung des Adapters 19. Unmittelbar nach der Aufteilung in die beiden Klebekapillaren 12, 12' schließen sich ein nach unten geneigter erster Verbindungsabschnitt 16 mit einem ersten Winkelabschnitt 15 und einem ersten Vertikalabschnitt 14 bzw. nach unten geneigter zweiter Verbindungsabschnitt 16' mit einem zweiten Winkelabschnitt 15' und einem zweiten Vertikalabschnitt 14' an. Die Vertikalabschnitte 14, 14' verlaufen im Wesentlichen parallel zu dem senkrechten Endabschnitt der Zuleitung 17. Die Öffnungen der Klebekapillaren 12, 12' zeigen also nach oben.

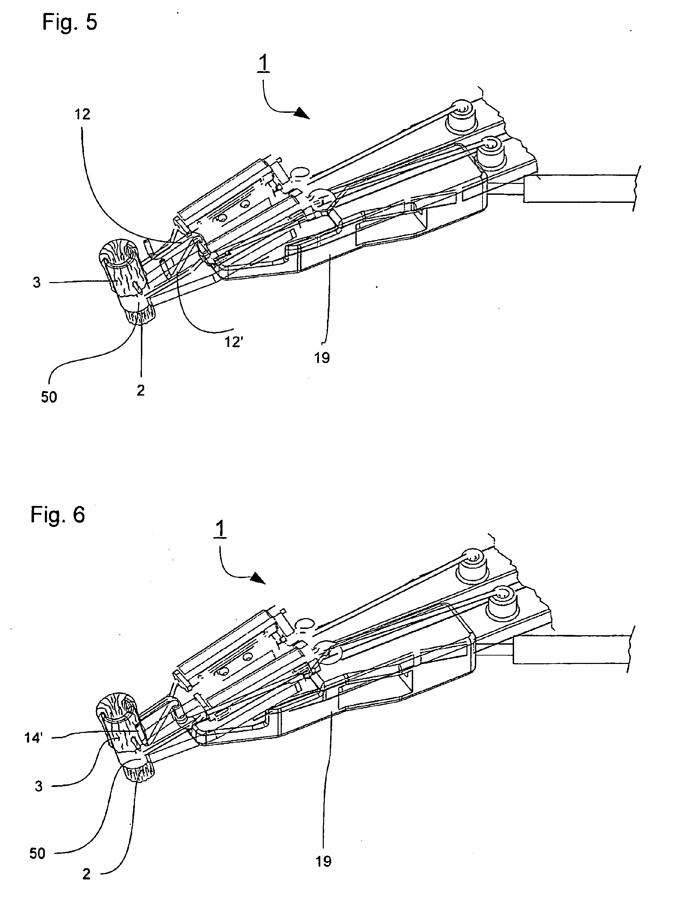

Der Klebeapplikator 10 ist ein Teil des elektrochirurgischen Instruments 1 und lässt sich mit diesem verbinden. Die Figuren 4 und 5 zeigen das elektrochirurgische Instrument 1 bei der Positionierung des Klebeapplikators 10. Das elektrochirurgische Instrument 1 umfasst eine Hülse 50 zur Aufnahme eines ersten Gefäßabschnitts 2 sowie zwei Hülsenbetätigungseinrichtungen 51, 51' (s. insbesondere Figur 4) mittels derer die zweigeteilte Hülse 50 in eine geöffnete und eine geschlossene Position bringbar ist. In der geschlossenen Position bildet die Hülse 50 eine Röhre, in die der erste Gefäßabschnitt 2 (z. B. ein Blutgefäß) eingeordnet werden kann. Nachdem der erste Gefäßabschnitt 2 durch die Hülse 50 eingeführt worden ist, lässt sich dieser derart umschlagen, dass das umgeschlagene Ende die Hülse 50 umschließt. Ein umgeschlagener Gewebeabschnitt 3 des ersten Gewebeabschnitts 2 ist in der Fig. 4 gezeigt. Die Innenfläche des umgeschlagenen Gewebeabschnitts 3 zeigt nach außen. In der in Fig. 4 gezeigten Position befindet sich der Klebeapplikator 10 in einer Aufnahmeposition, in der die Vertikalabschnitte 14, 14' von der Hülse 50 beabstandet sind. Der Vorgang des Umschlagens kann vorgenommen werden, ohne dass der Klebeapplikator 10 stört.

Fig. 5 zeigt ebenfalls den umgeschlagenen Gewebeabschnitt 3, der auf der Hülse 50 positioniert ist.

In Fig. 6 befindet sich der Klebeapplikator 10 in einer Applikationsposition, in der die Vertikalabschnitte 14, 14' auf dem umgeschlagenen Gewebeabschnitt 3 aufliegen und diesen zwischen den Vertikalabschnitten 14, 14' und der Hülse 50 fixieren. Die Vertikalabschnitte 14, 14' befinden sich wechselseitig zu der Hülse 50 und verlaufen im Wesentlichen parallel zum Hülsenkanal der Hülse 50. Die Öffnungen der Düsen der Klebekapillaren 12, 12' zeigen nach oben.

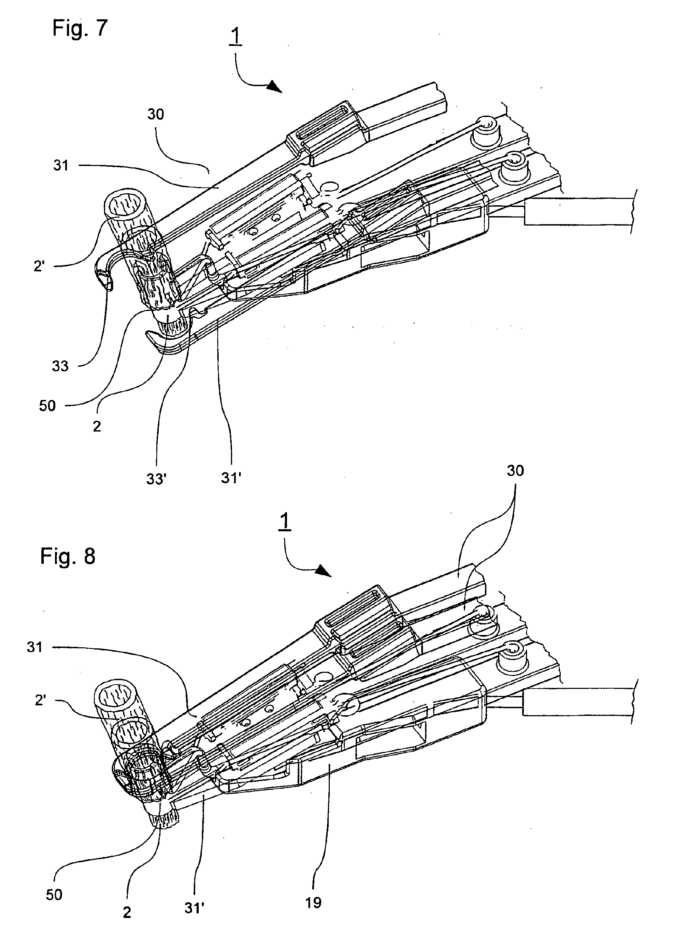

In Fig. 7 wird ein zweiter Gefäßabschnitt 2', der mit dem ersten Gefäßabschnitt 2 verbunden werden soll, über den umgeschlagenen Gewebeabschnitt 3 gezogen. Des Weiteren wird an dieser Stelle eine Kompressionszange 30 an dem Adapter 19 befestigt. Die Kompressionszange 30 hat eine erste Branche 31 und eine zweite Branche 31', die wechselseitig zur Hülse 50 angeordnet werden. Fig. 8 zeigt die Branchen 31, 31' im angeordneten Zustand, wobei sich diese in einer Kompressionsposition befinden, in der sie die Gefäßabschnitte 2, 2' federelastisch vorgespannt auf der Hülse 50 fixieren.

Beim Positionieren des zweiten Gefäßabschnitts 2' liegen die Klebekapillaren 12, 12' derart eng auf dem umgeschlagenen Gewebeabschnitt 3 an, dass der zweite Gefäßabschnitt 2' über diese übergestreift wird. Winkelabschnitte 15, 15' dienen als Begrenzung. Sie vereinfachen die Positionierung des zweiten Gefäßabschnitts 2' entlang einer Längsrichtung 56 der Hülse 50. Vorzugsweise wird der zweite Gefäßabschnitt 2' so lange über die Hülse 50 geschoben, bis das offene Ende an den Winkelabschnitten 15, 15' anstößt.

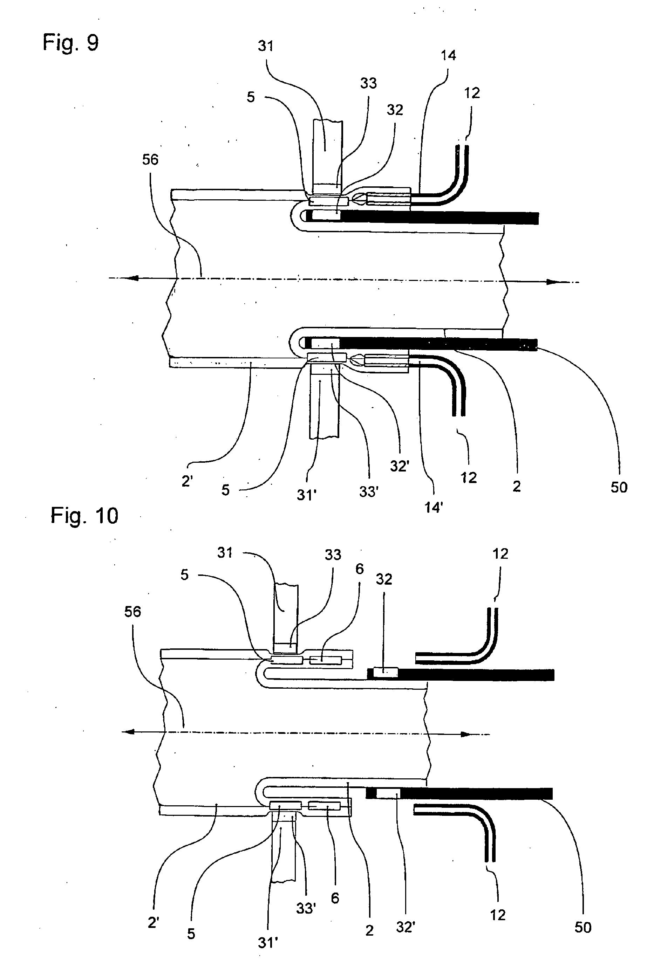

Fig. 9 zeigt einen schematischen Längsschnitt entlang der Längsrichtung 56 der Hülse 50 durch die Gefäßabschnitte 2, 2' und das elektrochirurgische Instrument 1, nämlich durch die Hülse 50, die Kompressionszange 30 und die Klebekapillaren 12, 12'. Die Gefäßabschnitte 2, 2' sind in das elektrochirurgische Instrument 1 eingelegt und die Kompressionszange 30 befindet sich in der Kompressionsposition. Ein wesentlicher Abschnitt des ersten Gefäßabschnitts 2 verläuft im Inneren der Hülse 50. Der umgeschlagene Gewebeabschnitt 3 liegt umgestülpt auf der Mantelfläche der Hülse 50 auf, wobei dessen Innenfläche nach außen zeigt. Auf diesem Abschnitt der Innenfläche des ersten Gefäßabschnitts 2 liegt ein Abschnitt der Innenfläche des zweiten Gefäßabschnitts 2' auf. Insgesamt erstrecken sich die Gefäßabschnitte 2, 2' entlang der Längsachse 56. Nahe dem oberen Ende der Hülse 50 wird diese durch die Kompressionszange 30 umschlossen. Die Branchen 31, 31' fixieren Abschnitte des ersten und zweiten Gefäßabschnitts 2, 2' auf der Hülse 50. Die erste Branche 31 weist an ihrer der Hülse 50 zugewandten Seite eine erste Außenelektrode 33 und die zweite Branche 31' eine zweite Außenelektrode 33' auf. Die Hülse 50 hat eine ringförmige Innenelektrode 32, die sich entlang der Mantelfläche erstreckt. Die Außenelektroden 33, 33' sind der Innenelektrode 32 gegenüberliegend angeordnet. Sie kontaktieren die überlappenden Gefäßabschnitte 2, 2' und können an einen HF-Generator angeschlossen werden, so dass eine HF-Spannung an diese angelegt werden kann. Ein applizierter HF-Strom durchfließt die Gefäßabschnitte 2, 2' und führt zu einem Verschmelzen des Gewebes. Es bildet sich eine ringförmige Gewebeverbindung 5 aus, die die Gefäßabschnitte 2, 2' verbindet.

Die Fig. 9 zeigt die Gefäßabschnitte 2, 2' mit der ringförmigen Gewebeverbindung 5. Jenseits der Gewebeverbindung 5 am offenen Ende der überlappenden Gefäßabschnitte 2, 2' sind die Klebekapillaren 12, 12' eingebracht. Über diese lässt sich ein Klebstoff einbringen, der die Gefäßabschnitte 2, 2' verbindet.

Fig. 10 zeigt die Klebeverbindung 6 zwischen den Gefäßabschnitten 2, 2'. Vorzugsweise wird der Klebstoff so zwischen die Gefäßabschnitte 2, 2' eingebracht, dass sich die Klebeverbindung 6 ebenfalls ringförmig entlang der Gewebeverbindung 5 erstreckt. Nach der Applikation des Klebstoffs lässt sich das elektrochirurgische Instrument 10, umfassend die Hülse 50 und die Klebekapillaren 12, 12' entfernen. Die Gewebeverbindung 5, die zeitlich vor der Klebeverbindung 6 hergestellt wird, verhindert das Eindringen des Klebstoffs in das Innere der verbundenen Gefäße. Die Klebeverbindung 6 verstärkt die Verbindung zwischen den Gefäßabschnitten 2, 2'. Aufgrund der Klebeverbindung 6 können die Gefäßabschnitte 2, 2' auch hohe Belastungen in Längsrichtung aufnehmen.

Auch wenn die vorliegende Erfindung hier in Verbindung mit einem elektrochirurgischen Instrument 1 beschrieben wurde, kann diese auch ohne das elektrochirurgische Instrument 1 sinnvoll verwandt werden.

In der vorhergehenden Beschreibung wird ein Klebeapplikator 10 beschrieben, der lediglich zwei Klebekapillaren 12, 12' umfasst. Theoretisch ist es möglich, mehrere Klebekapillaren 12, 12' an dem elektrochirurgischen Instrument 1 oder jeder anderen medizinischen Vorrichtung vorzusehen. Alternativ wäre es denkbar, eine Führung vorzusehen, mittels derer die Kapillaren 12, 12' um die Hülse 50 rotiert werden können, um eine durchgehende Klebeverbindung 6 herzustellen.

In dem vorhergehend beschriebenen Verfahren sollte eine Klebeverbindung 6 hergestellt werden, die sich ringförmig um die Hülse 50 erstreckt. Es kann jedoch ausreichen, dass eine entsprechende Klebeverbindung 6 lediglich abschnittsweise bereitgestellt wird.

### Bezugszeichenliste

- 1: elektrochirurgisches Instrument
- 2, 2': Gefäßabschnitt
- 3: umgeschlagener Gewebeabschnitt
- 5: Gewebeverbindung
- 6: Klebeverbindung
- 10: Klebeapplikator
- 12, 12': Klebekapillaren
- 14, 14': Vertikalabschnitt der Klebekapillaren
- 15, 15': Winkelabschnitt der Klebekapillaren
- 16, 16': Verbindungsabschnitt der Klebekapillaren
- 17: Zuleitung
- 18: Zuleitungsrohr
- 19: Adapter
- 20: Anschlussstück
- 21: Klebstoffanschluss
- 25: Treibmittelanschluss
- 30: Kompressionszange
- 31, 31': Branchen
- 32: Innenelektrode
- 33, 33': Außenelektrode
- 50: Hülse
- 51, 51': Hülsenbetätigungseinrichtung
- 56: Längsachse

## Patentansprüche

1. Vorrichtung zur Herstellung von Anastomosen zwischen einem ersten Hohlorgan (2) und einem zweiten Hohlorgan (2'), wobei die Hohlorgane (2, 2') jeweils eine Innenfläche haben, **dadurch gekennzeichnet, dass** die Vorrichtung von den verbundenen Hohlorganen (2, 2') abnehmbar ist, und dass die Vorrichtung umfasst:
- eine Hülse (50), über welche das erste Hohlorgan (2) so umstülpbar ist, dass mindestens ein Klebeverbundabschnitt der Innenfläche des ersten Hohlorgans (2) nach außen zu liegen kommt und auf welcher das zweite Hohlorgan (2') über dem ersten Hohlorgan (2) positionierbar ist;
- einen Klebeapplikator (10), der zur Applikation eines Klebstoffs auf den Klebeverbundabschnitt neben der Hülse (50) positionierbar ist.

2. Vorrichtung nach Anspruch 1, **gekennzeichnet durch** eine Führung, in der der Klebeapplikator (10) oder Teile des Klebeapplikators (10) zwischen einer Applikationsposition zur Applikation des Klebstoffs und einer Aufnahmeposition zur Aufnahme mindestens des ersten Hohlorgans **durch** die Hülse (50) verschiebbar angeordnet sind.

3. Vorrichtung nach Anspruch 2, **dadurch gekennzeichnet, dass** der Klebeapplikator (10) derart ausgebildet und angeordnet ist, dass in der Applikationsposition das erste Hohlorgan (2) zwischen dem Klebeapplikator (10) und der Hülse (50) fixierbar ist.

4. Vorrichtung nach einem der vorhergehenden Ansprüche, **dadurch gekennzeichnet, dass** der Klebeapplikator (10) mindestens einen Klebekanal (12, 12') umfasst, wobei zumindest ein Endabschnitt des Klebekanals (12, 12') zum Einbringen des Klebstoffs zwischen dem ersten und dem zweiten Hohlorgan (2, 2') parallel zur Hülse (50) angeordnet ist oder dort positionierbar ist.

5. Vorrichtung nach Anspruch 4, **dadurch gekennzeichnet, dass** der Klebeapplikator mindestens zwei Klebekanäle (12, 12') umfasst, die einen definierten Abstand zueinander aufweisen und wechselseitig neben der Hülse (50) positionierbar sind.

6. Vorrichtung nach Anspruch 4 oder 5, **dadurch gekennzeichnet, dass** der Klebekanal (12, 12') ein abgeflachtes Ende zum Applizieren des Klebstoffs umfasst.

7. Vorrichtung nach einem der Ansprüche 4 - 6, **dadurch gekennzeichnet, dass** der Klebeapplikator (10) mindestens einen Anschlag zur Positionierung des zweiten Hohlorgans (2') bezüglich der Hülse (50) umfasst.

8. Vorrichtung nach Anspruch 7, **dadurch gekennzeichnet, dass** der Klebekanal (12, 12') einen Winkelabschnitt (15, 15') als Anschlag für das zweite Hohlorgan (2') hat.

9. Vorrichtung nach einem der vorhergehenden Ansprüche, **dadurch gekennzeichnet, dass** die Hülse (50) an einer Betätigungseinrichtung (51, 51') gehalten, mindestens zweiteilig und derart zerlegbar ausgebildet ist, dass die Teile von einem Schließzustand zur Bildung eines geschlossenen Rohrabschnittes in einen Öffnungszustand zum Abnehmen vom Hohlorgan bringbar sind.

10. Vorrichtung nach einem der vorhergehenden Ansprüche, **gekennzeichnet durch** einen Kompressionsring, der die Hülse (50) zumindest abschnittsweise umgebend angeordnet ist oder entsprechend angeordnet werden kann, um in einer Kompressionsposition die Hohlorgane (2, 2') zwischen dem Kompressionsring und der Hülse (50) zu fixieren.

11. Vorrichtung nach Anspruch 10, **dadurch gekennzeichnet, dass** die Hülse (50) eine Innenelektrode (32) und der Kompressionsring eine Außenelektrode (33) zur Applikation eines HF-Stroms umfasst.

12. Vorrichtung nach einem der vorhergehenden Ansprüche, **dadurch gekennzeichnet, dass** der Klebeapplikator (10) abnehmbar mit der Vorrichtung verbunden ist.

13. Vorrichtung nach einem der vorhergehenden Ansprüche, **dadurch gekennzeichnet, dass** der Klebeapplikator (10) eine Zuleitung aus einem nicht adhäsiven Material umfasst.

14. Vorrichtung nach einem der vorhergehenden Ansprüche, **dadurch gekennzeichnet, dass** der Klebeapplikator (10) ein Klebstoffreservoir und einen Kompressor zur Applikation des Klebstoffs umfasst.

15. Vorrichtung nach Anspruch 14, **dadurch gekennzeichnet, dass** der Kompressor eine Spritze ist.

16. Vorrichtung nach Anspruch 14 oder 15, **dadurch gekennzeichnet, dass** der Klebeapplikator (10) ein Anschlussstück (20) für den Anschluss des Klebstoffreservoirs und des Kompressors an das proximale Ende der Klebekanäle (12, 12') des Klebeapplikators (10) umfasst.

17. Vorrichtung nach Anspruch 16, **dadurch gekennzeichnet, dass** das Anschlussstück (20) ein Y-förmiges Anschlussstück umfasst.

## Claims

1. Device for the creation of anastomoses between a first hollow organ (2) and a second hollow organ (2'), wherein the hollow organs (2, 2') respectively have an inside surface, **characterised in that** the device is removable from the connected hollow organs (2, 2') and that the device comprises:
• a sleeve (50), by means of which the first hollow organ (2) can be inverted in such a manner that at least one adhesive joint section of the inside surface of the first hollow organ (2) come to lie the outside and on which the second hollow organ (2') can be positioned above the first hollow organ (2);
• an adhesive applicator (10), which can be positioned next to the sleeve (50) for applying an adhesive to the adhesive joint section.

2. Device according to claim 1, **characterised by** a guide means, in which the adhesive applicator (10) or parts of the adhesive applicator (10) are arranged to be displaceable between an application position for applying the adhesive and a receiving position for receiving at least the first hollow organ by means of the sleeve (50).

3. Device according to claim 2, **characterised in that** the adhesive applicator (10) is configured and arranged in such a manner that in the application position the first hollow organ (2) can be secured between the adhesive applicator (10) and the sleeve (50).

4. Device according to one of the preceding claims, **characterised in that** the adhesive applicator (10) comprises at least one adhesive duct (12, 12'), wherein at least one end section of the adhesive duct (12, 12') is arranged parallel to the sleeve (50) or can be positioned there for introducing the adhesive between the first and the second hollow organ (2, 2').

5. Device according to claim 4, **characterised in that** the adhesive applicator comprises at least two adhesive ducts (12, 12'), which are spaced at a defined distance from one another and can be positioned on either side next to the sleeve (50).

6. Device according to claim 4 or 5, **characterised in that** the adhesive duct (12, 12') comprises a flattened end for applying the adhesive.

7. Device according to one of claims 4 - 6, **characterised in that** the adhesive applicator (10) comprises at least one stop for positioning the second hollow organ (2') in relation to the sleeve (50).

8. Device according to claim 7, **characterised in that** the adhesive duct (12, 12') has an angle section (15, 15') as stop for the second hollow organ (2').

9. Device according to one of the preceding claims, **characterised in that** held on an operating device (51, 51') the sleeve (50) is configured in at least two parts and is separable in such a manner that the parts can be moved from a closed state for forming a closed tube section into an open state for removing the hollow organ.

10. Device according to one of the preceding claims, **characterised by** a compression ring, which is arranged to surround the sleeve (50) at least in sections or can be arranged accordingly to secure the hollow organs (2, 2') in a compression position between the compression ring and the sleeve (50).

11. Device according to claim 10, **characterised in that** the sleeve (50) comprises an internal electrode (32) and the compression ring comprises an external electrode (33) for applying an HF current.

12. Device according to one of the preceding claims, **characterised in that** the adhesive applicator (10) is removably connected to the device.

13. Device according to one of the preceding claims, **characterised in that** the adhesive applicator (10) comprises a feed pipe made of a non-adhesive material.

14. Device according to one of the preceding claims, **characterised in that** the adhesive applicator (10) comprises an adhesive reservoir and a compressor for applying the adhesive.

15. Device according to claim 14, **characterised in that** the compressor is a syringe.

16. Device according to claim 14 or 15, **characterised in that** the adhesive applicator (10) comprises a connection piece (20) for connecting the adhesive reservoir and the compressor to the proximal end of the adhesive ducts (12, 12') of the adhesive applicator (10).

17. Device according to claim 16, **characterised in that** the connection piece (20) comprises a Y-shaped connection piece.

## Revendications

1. Dispositif permettant de réaliser des anastomoses entre un premier organe creux (2) et un deuxième organe creux (2'), les organes creux (2, 2') présentant chacun une surface intérieure, **caractérisé en ce que** le dispositif peut être retiré des organes creux (2, 2') reliés, et **en ce que** le dispositif comprend :
- un manchon (50), sur lequel le premier organe creux (2) peut être retourné de telle façon qu'au moins une portion de liaison par collage de la surface intérieure du premier organe creux (2) soit placée à l'extérieur, et sur lequel le deuxième organe creux (2') peut être positionné au-dessus du premier organe creux (2);
- un applicateur d'adhésif (10) qui peut être positionné à côté du manchon (50) en vue de l'application d'un adhésif sur la portion de liaison par collage.

2. Dispositif selon la revendication 1, **caractérisé en ce qu'**il présente un guide dans lequel l'applicateur d'adhésif (10) ou des parties de l'applicateur d'adhésif (10) sont disposés de manière à pouvoir être déplacés entre une position d'application, destinée à appliquer l'adhésif, et une position de réception, destinée à la réception au moins du premier organe creux par le manchon (50).

3. Dispositif selon la revendication 2, **caractérisé en ce que** l'applicateur d'adhésif (10) est réalisé et disposé de façon telle que, dans la position d'application, le premier organe creux (2) puisse être maintenu entre l'applicateur d'adhésif (10) et le manchon (50).

4. Dispositif selon l'une des revendications précédentes, **caractérisé en ce que** l'applicateur d'adhésif (10) comprend au moins un canal de collage (12, 12'), au moins une portion d'extrémité du canal de collage (12, 12') étant disposée parallèlement au manchon (50) ou pouvant être positionnée à cet endroit, pour introduire l'adhésif entre le premier et le deuxième organe creux (2, 2').

5. Dispositif selon la revendication 4, **caractérisé en ce que** l'applicateur d'adhésif comporte au moins deux canaux de collage (12, 12') qui présentent une distance définie l'un par rapport à l'autre et peuvent être positionnés en alternance à côté du manchon (50).

6. Dispositif selon la revendication 4 ou 5, **caractérisé en ce que** le canal de collage (12, 12') comporte une extrémité aplatie pour appliquer l'adhésif.

7. Dispositif selon l'une des revendications 4 à 6, **caractérisé en ce que** l'applicateur d'adhésif (10) comporte au moins une butée pour positionner le deuxième organe creux (2') par rapport au manchon (50).

8. Dispositif selon la revendication 7, **caractérisé en ce que** le canal de collage (12, 12') présente une portion coudée (15, 15') en tant que butée pour le deuxième organe creux (2').

9. Dispositif selon l'une des revendications précédentes, **caractérisé en ce que** le manchon (50) est maintenu sur un dispositif d'actionnement (51, 51'), est réalisé au moins en deux parties et peut être démonté de manière telle que les parties puissent être amenées d'un état de fermeture, destiné à former un tronçon de tube fermé, dans un état d'ouverture destiné à le retirer de l'organe creux.

10. Dispositif selon l'une des revendications précédentes, **caractérisé en ce qu'**il comporte une bague de compression qui est disposée de manière à entourer le manchon (50) au moins sur une partie, ou peut être disposée de manière correspondante, afin de maintenir, dans une position de compression, les organes creux (2, 2') entre la bague de compression et le manchon (50).

11. Dispositif selon la revendication 10, **caractérisé en ce que** le manchon (50) comprend une électrode interne (32) et la bague de compression comprend une électrode externe (33) pour l'application d'un courant HF.

12. Dispositif selon l'une des revendications précédentes, **caractérisé en ce que** l'applicateur d'adhésif (10) est relié de façon amovible au dispositif

13. Dispositif selon l'une des revendications précédentes, **caractérisé en ce que** l'applicateur d'adhésif (10) comprend une conduite d'amenée en matériau non adhésif.

14. Dispositif selon l'une des revendications précédentes, **caractérisé en ce que** l'applicateur d'adhésif (10) comprend un réservoir d'adhésif et un compresseur pour l'application de l'adhésif.

15. Dispositif selon la revendication 14, **caractérisé en ce que** le compresseur est une seringue.

16. Dispositif selon la revendication 14 ou 15, **caractérisé en ce que** l'applicateur d'adhésif (10) comprend une pièce de raccordement (20) pour le raccordement du réservoir d'adhésif et du compresseur à l'extrémité proximale des canaux de collage (12, 12') de l'applicateur d'adhésif (10).

17. Dispositif selon la revendication 16, **caractérisé en ce que** la pièce de raccordement (20) comprend une pièce de raccordement en forme de Y.
